(19) Europäisches Patentamt
European Patent Office
Office européen des brevets

(11) **EP 0 527 312 B1**

(12) **EUROPÄISCHE PATENTSCHRIFT**

(45) Veröffentlichungstag und Bekanntmachung des Hinweises auf die Patenterteilung:
**04.09.1996 Patentblatt 1996/36**

(51) Int. Cl.$^6$: **A61M 1/00**, A61B 17/32,
A61B 17/22

(21) Anmeldenummer: **92110205.9**

(22) Anmeldetag: **17.06.1992**

(54) **Spülkatheter**

Rinsing catheter

Cathéter de rinçage

(84) Benannte Vertragsstaaten:
**AT BE CH DE DK ES FR GB IT LI NL SE**

(30) Priorität: **14.08.1991 DE 4126886**

(43) Veröffentlichungstag der Anmeldung:
**17.02.1993 Patentblatt 1993/07**

(73) Patentinhaber: **CONVERGENZA AG**
**FL-9490 Vaduz (LI)**

(72) Erfinder:
• **Plechinger, Hans**
**W-8902 Neusäss (DE)**

• **Köhler, Josef, Dr.-Ing.**
**W-5100 Aachen (DE)**

(74) Vertreter: **Vetter, Ewald Otto, Dipl.-Ing. et al**
**Patentanwaltsbüro**
**Allgeier & Vetter**
**Postfach 10 26 05**
**86016 Augsburg (DE)**

(56) Entgegenhaltungen:
**EP-A- 0 175 096       EP-A- 0 390 993**
**EP-A- 0 442 579       WO-A-90/05493**
**US-A- 4 715 848**

## Beschreibung

Die Erfindung betrifft einen Spülkatheter zur Beseitigung von Feststoffen aus Körperorganen und Körperhohlorganen von Menschen und Tieren gemäß dem Oberbegriff von Anspruch 1.

Das Spülfluid ist vorzugsweise eine Flüssigkeit. Ein solcher Spülkatheter ist aus der EP 0 175 096 B1 (=US 4 690 672) bekannt. Dem Zufuhrlumen dieses Spülkatheters wird Flüssigkeit mit einem Druck von bis zu 30 bar zugeführt. Im Bereich der Düse hat die Flüssigkeit noch einen Druck von 5 bis 20 bar. Die Düse ist in einem zungenartig mit axialem Abstand über den Einlaß des Abfuhrlumens ragenden Abschnitt des Zufuhrlumens gebildet. Unter der Wirkung des aus der Düse austretenden Flüssigkeitsstrahles können in dem betreffenden Organ eines Menschen oder Tieres Feststoffe zertrümmert oder Ablagerungen aufgelöst werden. Die Trümmer werden unter der Wirkung eines Unterdruckes im Saugkanal, der an eine Saugquelle angeschlossen ist, in diesen Saugkanal eingesaugt, wobei der Transport in und durch den Saugkanal durch die aus der Düse sowie aus weiteren Öffnungen des Zufuhrlumens austretenden Flüssigkeitsstrahlen unterstützt wird. Die Düse kann axial verschiebbar angeordnet sein, damit ihr Abstand vom Einlaß des Abfuhrlumens der Größe des abzuführenden Feststoffes aus dem Organ angepaßt werden kann. Ein ähnlicher Spülkatheter, bei welchem jedoch die Düse des Zufuhrlumens die Form eines in Richtung zum Einlaß des Abfuhrlumens gebogenen Hakens hat, ist aus der US-PS 1 902 418 bekannt.

Durch die Erfindung soll die Aufgabe gelöst werden, einen Spülkatheter so auszubilden, daß mit ihm Ablagerungen, Wucherungen und Fremdmaterial aus Körperorganen und Körperhohlorganen wie z.B. Arterien, Venen, Herzhöhlen, Nasenhöhlen, Bronchien, Blasen usw. von Menschen und Tieren besser entfernt und abtransportiert werden können, ohne beim Zerkleinern und Abtransportieren des Materials Gefäßwände und Gewebe zu beschädigen. Auch sollen mit ihm Schleim und Fremdmaterial aus dem Trachea-Bronchialbaum abgesaugt sowie Ablagerungen und Thromben aus Blutgefäßen entfernt und abtransportiert werden können. Der Spülkatheter soll gemäß der Erfindung eine hohe Betriebssicherheit, eine einfache Handhabung und einen so geringen Herstelllungspreis haben, daß er auch als Einmalartikel für die rationelle Serienfertigung geeignet ist.

Diese Aufgabe wird gemäß der Erfindung durch die kennzeichnenden Merkmale von Anspruch 1 gelöst.

Durch den Diffusor des erfindungsgemäßen Spülkatheters wird am Einlaß des Abfuhrlumens eine so starke Saugwirkung erzielt, daß auch mit einem kleinvolumigen Spülkatheter ein Zertrümmern und Abtransportieren von Feststoffen aus dem betreffenden Organ möglich ist, und in dem Organ ein großer Bereich behandelt werden kann. Ein wesentlicher Vorteil der Erfindung ist, daß normalerweise an das Abfuhrlumen keine Saugquelle angeschlossen zu werden braucht, und für seltene Bedarfsfälle die Saugquelle eine wesentlich geringere Saugleistung haben kann als bei bekannten Spülkathetern. Das Spülfluid, vorzugsweise eine Flüssigkeit, kann dem Zufuhrlumen mit einem Druck von über 150 bar zugeführt werden.

Beim Spülkatheter nach der Erfindung ist die Düse so ausgebildet, und sie hat einen solchen Abstand vom Einlaß des Abfuhrlumens, daß sich in dem dazwischenliegenden Fluidstrahl der Düse Unterdrücke ohne Rückströmungen einstellen. Die Düse bildet zusammen mit dem Abfuhrlumen, insbesondere durch den im Abfuhrlumen enthaltenen Diffusor, einen Strahlapparat mit besonders guter Saugwirkung, weil der Fluidstrahl der Düse bis zum Einlaß des Abfuhrlumens nicht so weit aufgeweitet wird, daß er an dem Einlaß vorbeiströmt. Durch den scharfen Fluidstrahl oder Flüssigkeitsstrahl, der nach Art eines Strahlsaugers aus der Düse austritt und von dem Einlaß des Abfuhrlumens aufgefangen wird, werden folgende Wirkungen erzielt:

a) eine Saugwirkung
b) eine Zerkleinerungswirkung (Schreddern, Zertrümmern und Auflösen) und
c) eine Transportwirkung (Rücktransport des Spülfluides zusammen mit dem vom Spülfluid im Organ mitgerissenen Material.

Das Ansaugen dient der Zufuhr von Material aus dem Organ in die Nähe des Zerkleinerungsbereiches am Einlaß des Abfuhrlumens. Nach der Zerkleinerung wird das Gemisch aus Spülfluid und zerkleinertem Material durch das Abfuhrlumen aus dem Organ herausbefördert. Mit dem Spülkatheter nach der Erfindung können bei nur geringer Belastung und ohne unzulässiges Risiko für den Patienten in seinem Gefäßsystem Verengungen und Verschlüsse beseitigt werden, ohne daß Rückstände verbleiben. Als wichtigste Anwendungsbereiche stehen das Herz und die Extremitäten zunächst im Vordergrund. Der Spülkatheter ist jedoch auch zur intraoperativen Behandlung in Hohlorganen, an Zähnen und an der Haut geeignet. Als Spülfluid dient vorzugsweise Flüssigkeit, z.B. Suspensionen, jedoch können je nach Anwendungszweck auch Gase verwendet werden. Der Spülkatheter kann von Hand, teilautomatisiert oder vollautomatisch benutzt werden.

Gemäß der Erfindung kann der Spülkatheter folgende Merkmale aufweisen: Das Verhältnis des Strömungsdurchmessers der Düse zu dem Strömungsdurchmesser des Mischrohres beträgt vorzugsweise 0,2 bis 0,7; der Abstand der Düse vom stromaufwärtigen Ende des Mischrohres, gebildet durch den Einlaß des Abfuhrlumens, beträgt vorzugsweise das 0,3-fache bis 1,5-fache des Strömungsdurchmessers des Mischrohres; die Länge des zylindrischen Mischrohres beträgt vorzugsweise das 2,5-fache bis 8,2-fache seines Strömungsdurchmessers; der Öffnungswinkel des Diffusors beträgt vorzugsweise 2° bis 30°; die Düse kann an einem hakenartigen Endabschnitt des Zufuhrlumens gebildet sein oder das vordere Ende des Spülkatheters kann wie ein symmetrischer oder unsymmetrischer Fischmaulkopf ausgebildet sein; das Mischrohr und der Diffusor bestehen vorzugsweise aus einem Stück; das distale Ende des Spülkatheters enthält vorzugsweise ein zusätzliches Gerät, beispielsweise ein Schneidelement für Schneidarbeiten im Organ, ein Druckmeßgerät, ein Ultraschallgerät oder/und ein Lasergerät.

Die Erfindung wird im folgenden mit Bezug auf die Zeichnungen anhand mehrerer bevorzugter Ausführungsformen als Beispiele beschrieben. Darin zeigen

Fig. 1 einen Längsschnitt durch eine Arterie mit einem eingesetzten Spülkatheter nach der Erfindung,

Fig. 2 einen Längsschnitt des Spülkatheters von Fig. 1 in anderer Darstellung,

Fig. 3 einen Querschnitt längs der Ebene III-III von Fig. 2,

Fig. 4 einen Querschnitt längs der Ebene IV-IV von Fig. 2,

Fig. 5 einen Querschnitt einer weiteren Ausführungsform eines Spülkatheters nach der Erfindung längs der Ebene III-III von Fig. 2,

Fig. 6 einen Querschnitt der Ausführungsform von Fig. 5 längs der Ebene VI-VI von Fig. 2,

Fig. 7 einen Längsschnitt eines vorderen Endabschnittes einer weiteren Ausführungsform eines Spülkatheters nach der Erfindung,

Fig. 8 einen vorderen Endabschnitt im Längsschnitt einer nochmals weiteren Ausführungsform eines Spülkatheters nach der Erfindung, und

Fig. 9 die Anwendung des Spülkatheters nach den Fig. 1 bis 4 bei vollautomatischem Betrieb.

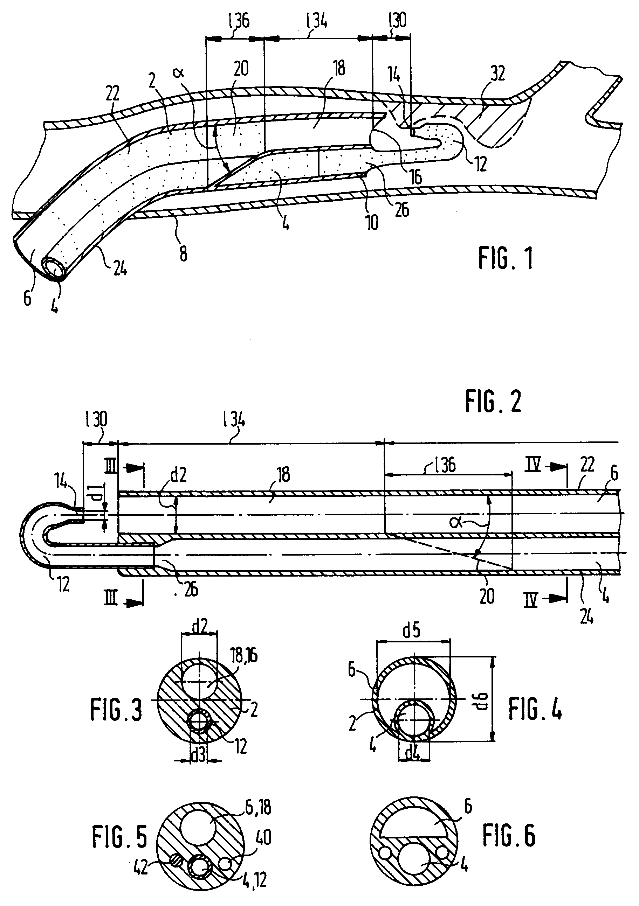

Die Fig. 1 bis 4 zeigen einen Spülkatheter 2 mit zwei Lumen 4 und 6, welcher in ein Körpergefäß 8, beispielsweise eine Arterie, eingeführt ist. Das eine Lumen 4 ist ein Zufuhrlumen zur Zufuhr von Spülfluid, insbesondere einer Flüssigkeit, zum vorderen Ende 10 des Spülkatheters 2, wo das Zufuhrlumen 4 mit einem um 180° zurückgebogenen Krümmer 12 und an dessen Ende mit einer Düse 14 versehen ist. Die Spülflüssigkeit strömt von dem Zufuhrlumen 4 aus der Düse 14 in Form eines scharfen Strahles axial in den stirnseitigen Einlaß 16 am vorderen Ende des Abfuhrlumens 6. Der Druck des Spülfluides an der Düse 14 kann bis zu 150 bar betragen. Das Abfuhrlumen 6 besteht nacheinander aus folgenden Abschnitten: einem den Einlaß 16 bildenden zylindrischen Mischrohr 18, einem sich stromabwärts anschließenden Diffusor 20 und einem Rückführkanal 22 oder einer Rückführleitung 22. Das Zufuhrlumen 4 und das Abfuhrlumen 6 sind achsparallel zueinander versetzt ineinander angeordnet. Sie können aus einem einzigen Stück oder mehreren Stücken bestehen. Vorzugsweise bestehen das Mischrohr 18 und der Diffusor 20 zusammen aus einem einzigen Stück, und der Krümmer 12 und die Düse 14 bestehen aus einem einzigen Stück. Der einstückige Abschnitt "Mischrohr 18 und Diffusor 20" ist an die Rückführleitung 22 angeschlossen, vorzugsweise angeklebt oder angeschweißt. Das einstückige Teil "Krümmer 12 und Düse 14" ist in den eine Zufuhrleitung 24 bildenden Abschnitt des Zufuhrlumens 4 eingesetzt, vorzugsweise eingeklebt oder eingeschweißt. Die Düse 14 ist durch einen in Strömungsrichtung im Durchmesser reduzierten Abschnitt gebildet. Der Krümmer-Düsen-Teil 12, 14 enthält vorzugsweise stromaufwärts des Krümmers 12 einen zusätzlichen, in Strömungsrichtung im Querschnitt verjüngten Düsenabschnitt 26. Durch diese zweimalige Querschnittsverringerung im ersten Düsenabschnitt 26 und in der folgenden Düse 14 wird ein sehr dünner, scharfer Fluidstrahl gebildet. Der Abstand 130 zwischen dem Austrittsende der Düse 14 und dem Einlaß 16 des Abfuhrlumens 6 bzw. dessen Mischrohres 18 ist so groß, daß sich der scharfe Fluidstrahl auf diesem Abstand I 30 nicht auflöst, sondern dazwischenliegendes Gewebe 32 des Gefäßes 8 schreddern oder zertrümmern und die zertrümmerten Stoffteilchen in den Einlaß 16 treiben kann. Das Gefäß 8 kann auch als Hohlorgan bezeichnet werden. Der Spülkatheter kann jedoch auch in Vollorgane von Menschen oder Tieren eingeführt werden. Die Länge l 34 des Mischrohres und die Länge l 36 des Diffusors sowie der Öffnungswinkel des Diffusors 20 sind so aufeinander und auf die Stärke des aus der Düse 14 austretenden Fluidstrahles abgestimmt, daß die Wirkung des Diffusors 20 durch das Spülfluid im Mischrohr 18 hindurch eine Verstärkung der Saugwirkung des Strahles bewirkt, welcher von der Düse 14 in den Einlaß 16 des Mischrohres 18 strömt.

Der Impuls des aus der Düse 14 austretenden und in den Einlaß 16 strömenden Spülfluidstrahles, welcher beispielsweise aus einer isotonischen Kochsalzlösung und/oder Luft besteht und ein Stromvolumen $Q_t$ hat, wird durch Reibung und Turbulenz teilweise auf das umgebende Medium innerhalb des Abstandes 130 übertragen und bewirkt das Ansaugen eines Saugstromvolumens $Q_s$. Dieser Saugeffekt wird dazu verwendet, Material aus dem Gefäß 8 oder einem anderen Hohlorgan zu entfernen, beispielsweise Schleim aus Bronchien oder Thromben 32 aus Blutgefäßen 8. Der Spülfluidstrahl der Düse 14 kann zusätzlich zur Erzeugung dieser Saugwirkung auch dazu benutzt werden, festes Material in dem betreffenden Organ 8 zu zertrümmern oder zu zerschreddern oder aufzulösen. Der insgesamt durch das Abfuhrlumen 6 des Katheters 2 aus dem Körper herausgeführte Volumenstrom hat dann das Volumen $Q_g = Q_t + Q_s$. In den Zeichnungen bedeuten d1 der Durchmesser des vorzugsweise kreisrunden Innenquerschnittes der Düse 14; d2 der Durchmesser des vorzugsweise kreisrunden Strömungsquerschnittes des Einlasses 16 und des Mischrohres 18; d3 der Durchmesser des vorzugsweise kreisrunden inneren Strömungsquerschnittes des Krümmers 12 und des unmittelbar stromaufwärts davon in der Schnittebene III-III gelegenen Strömungsquerschnittes des Zufuhrlumens 4, stromabwärts des ersten Düsenabschnittes 26; d4 der Durchmesser des kreisrudnen Strömungsdurchmessers des Zufuhrlumens 4 in seinem eine Zufuhrleitung 24 bildenden Abschnitt stromaufwärts des ersten Düsenabschnittes 26; d5 der Innendurchmesser des kreisrunden Strömungsquerschnittsteils des Abfuhrlumens 6 in seinem eine Rückführleitung bildenden Abschnitt 22 stromabwärts des Diffusors 20; d6 der Außendurchmesser des außen vorzugsweise kreisrunden Spülkatheters 2. Da das Zufuhrlumen 4 achsparallel versetzt innerhalb des Abfuhrlumens 6 angeordnet ist, hat das Zufuhrlumen 4 einen kreisrunden Innenquerschnitt, das Abfuhrlumen 6 jedoch einen halbmondförmigen Innenquerschnitt, entsprechend Fig. 4 an der Schnittebene IV-IV von Fig. 2.

Der Spülkatheter dieser Art hat den Vorteil, daß er auch dann noch eine sehr gute Wirkung hat, wenn er in sehr kleinen Abmessungen hergestellt wird. Dabei sind folgende Größenverhältnisse wichtig: das Verhältnis d1/d2 des inneren Durchmessers d1 der Düse 14 zum inneren Durchmesser d2 des Mischrohres 18 und seines Einlasses 16 sollte im Bereich von 0,2 bis 0,7 liegen; das Verhältnis l 30/d2 des Abstandes l 30 der Düse 14 vom Einlaß 16 des Mischrohres 18 zum Innendurchmesser d2 des Mischrohres 18 und seines Einlasses 16 sollte zwischen 0,3 bis 1,5 liegen; das Verhältnis l 34/d2 von Länge l 34 zum Innendurchmesser d2 des Mischrohres 18 sollte zwischen 2,5 und 8,2 liegen; und der Öffnungswinkel $\alpha$ des Diffusors 20 sollte zwischen 2° und 30° betragen.

Die in den Fig. 5 und 6 dargestellte weitere Ausführungsform eines Spülkatheters zeigt, daß er auch mehr als zwei Lumen 4 und 6 enthalten kann, beispielsweise zusätzlich ein Lumen 40 zur Druckmessung in dem zu behandelnden Organ 8 und ein Lumen 42, in welchem zusätzliche Behandlungsgeräte am vorderen Ende 10 angeordnet werden können, beispielsweise ein Druckmeßelement, ein Ultraschallkopf oder ein Laserkopf.

Funktion: Der Spülkatheter bildet ein Strahl-Saug-Gerät. Der Diffusor 20 bewirkt einen Druckrückgewinn im Abfuhrlumen 6 und unterstützt damit die Rückführung des Spülfluides und die Abfuhr des im Spülfluid mitgeführten Materials aus dem behandelten Organ 8. Der Diffusor 20 bewirkt eine Reduzierung des Druckverlustes im Abfuhrlumen 6; ferner einen Anstieg des Saugstromverhältnisses $q = Q_s/Q_t$; und einen Anstieg des Wirkungsgrades. Das Diffusor-Querschnittsverhältnis von Diffusorquerschnitt an seinem stromaufwärtigen Anfang zum Diffusorquerschnitt an seinem stromabwärtigen Ende hat einen entscheidenden Einfluß auf die Saugcharakteristik und den Wirkungsgrad. Der Spülfluidstrahl zwischen der Düse 14 und dem Einlaß 16 des Mischrohres 18 "reißt" aufgrund seiner Reibung aus seiner Umgebung Material mit und erzeugt dadurch im Bereich des Abstandes l 30 einen Unterdruck. Der Druck am Einlaß 16 ist über die Spülfluidströmung im Abfuhrlumen 6 im Gleichgewicht mit dem Druck am stromabwärtigen Ende des Abfuhrlumens 6, wo das vom Spülfluid mitgeführte Material in einen Auffangbehälter gelangt. Der Auffangbehälter ist normal belüftet und hat damit Atmosphärendruck. Der Druck am Einlaß 16 des Mischrohres 18 liegt um die Summe aller Druckverluste und Druckrückgewinne über oder unter dem Atmosphärendruck. Damit er unter dem Atmosphärendruck liegen kann, muß der Druckrückgewinn im Abfuhrlumen 6 hinreichend groß sein. Der in dem zu behandelnden Organ 8 erforderliche Unterdruck entsteht in der genannten Weise durch den Spülfluidstrahl der Düse 14 und durch den Diffusor 20. Es entsteht zwar ein Druckrückgewinn durch die Querschnittserweiterung des Spülfluidstrahles der Düse 14 bei der Aufweitung des Spülfluidstrahles im Übergang von der im Querschnitt kleinen Düse 14 in das im Querschnitt größere Mischrohr 18, jedoch ist dieser Druckrückgewinn zur Erzielung einer zufriedenstellenden Saugwirkung in dem behandelten Organ 8 nicht ausreichend. Eine ausreichene Saugwirkung wird erst durch die zusätzliche Wirkung des Diffusors 20 erreicht.

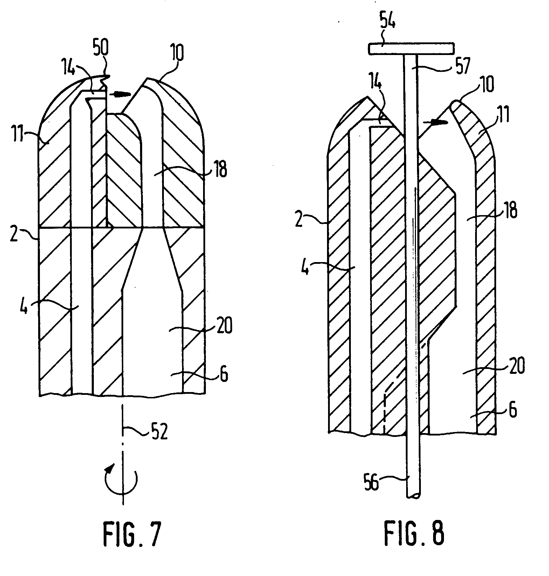

Bei den Ausführungsformen der Fig. 7 und 8 sind den Fig. 1 bis 6 funktionsmäßig entsprechende Teile mit gleichen Bezugszahlen versehen. Der wesentliche Unterschied besteht darin, daß bei den Ausführungsformen nach den Fig. 7 und 8 die Düse 14 nicht am Ende eines Krümmers 12 angeordnet ist, sondern in einem symmetrisch oder unsymmetrisch fischmaulartigen Endabschnitt 11 des Spülkatheters 2. Fig. 7 zeigt, daß am distalen Ende des Spülkatheters 2 eine Schneide 50 zum Schneiden von Material in dem zu behandelnden Organ 8 vorgesehen sein kann. Für den Schneidvorgang kann der Spülkatheter 2 beispielsweise um seine zentrale Symmetrieachse 52 gedreht werden. Aus der Ausführungsform von Fig. 8 ist ersichtlich, daß als Schneidwerkzeug 54 auch ein Rotationsmesser vorgesehen sein kann, welches am vorderen Ende 10 einer durch den Katheter 2 axial hindurchgeführten Welle 56 befestigt ist. An der Welle 56 könnte an ihrem vorderen Ende 57 anstelle eines Schneidwerkzeuges 54 auch ein anderes Gerät befestigt sein, beispielsweise ein optisches Sichtgerät, ein Ultraschallgerät, ein Lasergerät oder dergleichen.

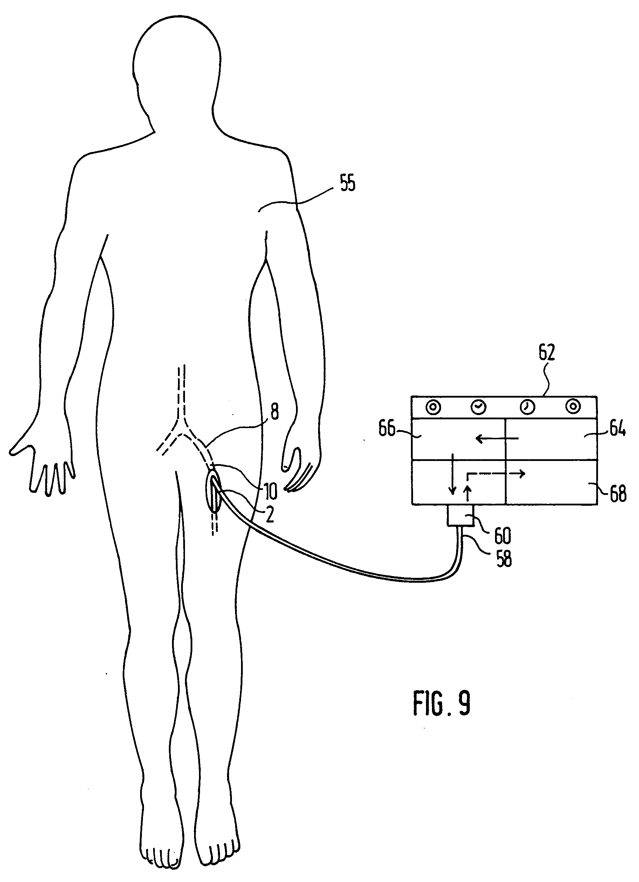

Fig. 9 zeigt die praktische Anwendung des Spülkatheters 2, welcher mit seinem vorderen Ende 10 in eine Arterie 8 einer Person 55 eingesetzt ist. Das hintere Ende 58 des Spülkatheters 2, oder dessen Zuleitungen, ist über eine Kupplung 60 an ein Gerät 62 zur automatischen Organbehandlung angeschlossen. Das Gerät 62 enthält beispielsweise einen Vorratsbehälter 64 für das Spülfluid, eine Pumpe 66, welche das Spülfluid von dem Behälter 64 in das Zufuhrlumen 4 des Katheters 2 pumpt, und einen Sammelbehälter 68, in welchem das vom Spülfluid über das Abfuhrlumen 6 aus dem Organ 8 gespülte Material aus dem Spülfluid ausgeschieden und gesammelt wird.

**Patentansprüche**

1. Spülkatheter zur Beseitigung von Feststoffen aus Körperorganen und Körperhohlorganen (8) von Menschen und Tieren, mit mindestens zwei Lumen (4, 6) von welchen ein erstes Lumen (4) ein Zufuhrlumen zur Zufuhr von Spülfluid von einer Hochdruckfluidquelle (66) in das betreffende Organ (8) und ein zweites Lumen (6) ein Abfuhrlumen zur Abfuhr von Spülfluid und von, vom Spülfluid mitgenommenen Feststoffteilchen aus dem Organ (8) ist, mit einer den Auslaß des Zufuhrlumens (4) bildenden Düse (14), welche im Abstand (l 30) gegenüber dem Einlaß (16) des Abfuhrlumens (6) angeordnet ist und in diesen Einlaß (16) einen starken, scharf gebündelten Spülfluidstrahl schickt, der zwischen der Düse (14) und dem Einlaß (16) gelegenes Material des Organs (8) ansaugt, gegebenenfalls zertrümmert oder auflöst, und über das Abfuhrlumen (6) abführt,
**dadurch gekennzeichnet,**
daß das Abfuhrlumen (6) in Strömungsrichtung nacheinander folgende Abschnitte aufweist: ein Mischrohr (18), dessen Einlaß der Einlaß (16) des Abfuhrlumens (6) ist und in welchem sich die vom Spülfluidstrahl angesaugten Feststoffteilchen im Spülfluid verteilen können, einen Diffusor (20) und einen Abfuhrkanal (22), wobei die Länge (l 34) des Mischrohres (18) und die Abmessungen (l 36, $\alpha$) des Diffusors so bemessen sind, daß der Diffusor (20) - im Vergleich zu einem zylindrischen Kanalabschnitt - eine Verstärkung der Saugwirkung des Spülfluidstrahles am Einlaß des Mischrohres (18) bewirkt.

2. Spülkatheter nach Anspruch 1,
**dadurch gekennzeichnet,**
daß das Verhältnis (d1/d2) des Strömungsdurchmessers (d1) der Düse an ihrem Auslaßende zu dem Strömungsdurchmesser (d2) des Mischrohres 0,2 bis 0,7 beträgt.

3. Spülkatheter nach Anspruch 1 oder 2,
**dadurch gekennzeichnet,**
daß der Abstand (l 30) der Düse (14) vom Einlaß (16) des Mischrohres (18) das 0,3-fache bis 1,5-fache des Strömungsdurchmessers (d2) des Mischrohres (18) beträgt.

4. Spülkatheter nach einem der Ansprüche 1 bis 3,
**dadurch gekennzeichnet,**
daß die Länge des im wesentlichen zylindrischen Mischrohres (18) das 2,5-fache bis 8,2-fache seines Strömungsdurchmessers (d2) beträgt.

5. Spülkatheter nach einem der Ansprüche 1 bis 4,
**dadurch gekennzeichnet,**
daß der Öffnungswinkel ($\alpha$) des Diffusors 2° bis 30° beträgt.

6. Spülkatheter nach einem der Ansprüche 1 bis 5,
**dadurch gekennzeichnet,**
daß das Mischrohr (18) und der Diffusor (20) aus einem Materialstück bestehen, welches an dem stromabwärts davon gelegenen Abschnitt des Abfuhrlumens (6) befestigt ist.

7. Spülkatheter nach einem der Ansprüche 1 bis 6,
**dadurch gekennzeichnet,**
daß am vorderen Ende (10) ein zusätzliches Gerät - insbesondere ein Schneidelement (50) für Schneidarbeiten in dem behandelten Organ (8), ein Druckmeßelement, ein Ultraschallgerät oder/und Lasergerät (42, 54) - angeordnet ist.

8. Spülkatheter nach einem der Ansprüche 1 bis 7,
**dadurch gekennzeichnet,**
daß die Düse (14) an einem hakenartigen Endabschnitt (12, 26) des Zufuhrlumens (4) für das Spülfluid gebildet ist.

9. Spülkatheter nach einem der Ansprüche 1 bis 7,
**dadurch gekennzeichnet,** daß die Düse (14) in einem vorderen, in das Organ (8) einführbaren, symmetrisch oder unsymmetrisch fischmaulartigen Endabschnitt (11) des Spülkatheters (2) angeordnet ist.

## Claims

1. Rinsing catheter for the removal of substances from organs of the body and hollow organs of the body (8) in humans and animals, comprising at least two lumina (4, 6), of which the first lumen (4) is a supply lumen for the supply of rinsing fluid from a high pressure fluid source (66) into the organ (8) in question and a second lumen (6) which is a discharge lumen for removing rinsing fluid and particles of solid matter carried in the rinsing fluid from the organ (8), comprising a nozzle (14) which forms the outlet of the supply lumen (4) and which is fitted in the space (l 30) facing the inlet (16) of the discharge lumen (6) and sends a powerful, sharply focused jet of rinsing fluid into this inlet (16) which draws in, and where appropriate shatters or disperses matter located between the nozzle (14) and the inlet (16) from the organ (8) and carries it away via the discharge lumen (6),
**characterised in that**
the discharge lumen (6) has the following successive parts in the direction of flow: a mixer tube (18), the inlet of which is the inlet (16) of the discharge lumen (6) and in which the particles of solid matter sucked up by the jet of rinsing fluid can be dispersed in the rinsing fluid, a diffuser (20) and a discharge duct (22), in which the length (l 34) of the mixer tube (18) and the dimensions (l 36, $\alpha$) of the diffuser are designed so that the diffuser (20) - compared with a cylindrical duct section - produces an intensification of the suction effect of the jet of rinsing fluid at the inlet of the mixer tube (18).

**2.** Rinsing catheter according to Claim 1, **characterised in that** the ratio (d1/d2) of the flow diameter (d1) of the nozzle at its outlet end to the flow diameter (d2) of the mixer tube is 0.2 to 0.7.

**3.** Rinsing catheter according to Claim 1 or 2, **characterised in that** the distance (l 30) of the nozzle (14) from the inlet (16) of the mixer tube (18) is 0.3 to 1.5 times the flow diameter (d2) of the mixer tube (18).

**4.** Rinsing catheter according to one of the Claims 1 to 3, **characterised in that** the length of the essentially cylindrical mixer tube (18) is 2.5 to 8.2 times its flow diameter (d2).

**5.** Rinsing catheter according to one of the Claims 1 to 4, **characterised in that** the aperture angle ($\alpha$) of the diffuser is 2° to 30°.

**6.** Rinsing catheter according to one of the Claims 1 to 5, **characterised in that** the mixer tube (18) and the diffuser (20) consist of one piece of material which is attached to the section of discharge lumen (6) which is located downstream of it.

**7.** Rinsing catheter according to one of the Claims 1 to 6, **characterised in that** an additional device - especially a cutting device (5) for carrying out cutting operations in the organ being treated (8), a pressure measuring device, an ultrasound device and/or a laser device (42, 54) - is fitted at the leading end (10).

**8.** Rinsing catheter according to one of the Claims 1 to 7, **characterised in that** the nozzle (14) is formed on a hook-shaped end section (12, 26) of the supply lumen (4) for the rinsing fluid.

**9.** Rinsing catheter according to one of the Claims 1 to 7, **characterised in that** the nozzle (14) is fitted in a symmetrical or asymmetrical fish-mouth shaped leading end section (11) of the rinsing catheter (2) which can be introduced into the organ (8).

**Revendications**

**1.** Cathéter de rinçage ou de lavage pour éliminer des matières solides d'organes corporels ou d'organes corporels creux (8) de l'homme et d'animaux, comprenant au moins deux lumières (4, 6) dont une première (4) est une lumière d'amenée pour amener du fluide de lavage à partir d'une source de fluide à haute pression (66) dans l'organe (8) concerné et dont la deuxième (6) est une lumière d'évacuation pour évacuer le fluide de lavage et les fragments de matière solide entraînés par ce fluide hors de l'organe (8), ainsi qu'une buse (14), formant la sortie de la lumière d'amenée (4), qui est disposée à distance (l 30) en regard de l'admission (16) de la lumière d'évacuation (6) et qui envoie dans cette admission (16) un jet de fluide de lavage puissant et fortement concentré, jet qui aspire la matière de l'organe (8) située entre la buse (14) et l'admission (16), fragmente ou dissout éventuellement cette matière et l'évacue à travers la lumière d'évacuation (6), caractérisé en ce que la lumière d'évacuation (6) comporte, l'une après l'autre, dans la direction d'écoulement, les parties suivantes: un tube mélangeur (18) dont l'entrée constitue l'admission (16) de la lumière d'évacuation (6) et à l'intérieur duquel les fragments de matière solide aspirés par le jet de fluide de lavage peuvent se répartir dans le fluide, un diffuseur (20) et un canal d'évacuation (22), la longueur (l 34) du tube mélangeur (18) et les dimensions (l 36, $\alpha$) du diffuseur étant choisies de manière que le diffuseur (20) - en comparaison avec une portion de canal cylindrique - provoque une amplification de l'effet d'aspiration du jet de fluide de lavage à l'entrée du tube mélangeur (18) .

**2.** Cathéter de lavage selon la revendication 1, caractérisé en ce que le rapport (d1/d2) du diamètre d'écoulement (d1) de la buse à son extrémité de sortie au diamètre d'écoulement (d2) du tube mélangeur est de 0,2 à 0,7.

**3.** Cathéter de lavage selon la revendication 1 ou 2, caractérisé en ce que la distance (l 30) qui sépare la buse (14) de l'entrée (16) du tube mélangeur (18) correspond à 0,3 fois à 1,5 fois le diamètre d'écoulement (d2) du tube mélangeur (18).

**4.** Cathéter de lavage selon une des revendications 1 à 3, caractérisé en ce que la longueur du tube mélangeur (18), qui est essentiellement cylindrique, correspond à 2,5 fois à 8,2 fois son diamètre d'écoulement (d2).

**5.** Cathéter de lavage selon une des revendications 1 à 4, caractérisé en ce que l'angle d'ouverture ($\alpha$) du diffuseur est de 2° à 30°.

**6.** Cathéter de lavage selon une des revendications 1 à 5, caractérisé en ce que le tube mélangeur (18) et le diffuseur (20) sont faits d'une seule pièce fixée à la partie située en aval d'eux de la lumière d'évacuation (6).

7. Cathéter de lavage selon une des revendications 1 à 6, caractérisé en ce qu'un appareil supplémentaire est disposé à l'extrémité avant (10) du cathéter, en particulier un élément coupant (50) pour effectuer des résections dans l'organe (8) traité, un élément de mesure de pression, un appareil à ultrasons et/ou un appareil laser (42, 54).

8. Cathéter de lavage selon une des revendications 1 à 7, caractérisé en ce que la buse (14) est formée sur une portion terminale (12, 26) semblable à un crochet de la lumière d'amenée (4) pour le fluide de lavage.

9. Cathéter de lavage selon une des revendications 1 à 7, caractérisé en ce que la buse (14) est agencée dans une portion terminale avant (11) du cathéter de lavage (2), portion qui peut être introduite dans l'organe (8) et possède une configuration symétrique ou dissymétrique "en bouche de poisson".

FIG. 1

FIG. 2

FIG.3

FIG. 4

FIG.5

FIG.6

FIG. 7

FIG. 8

FIG. 9